# EUROPÄISCHE PATENTSCHRIFT

(11) **EP 1 069 117 B1**
(45) Veröffentlichungstag und Bekanntmachung des Hinweises auf die Patenterteilung: **15.01.2003**
(21) Anmeldenummer: 00113134.1
(22) Anmeldetag: 29.06.2000
(51) Int. Cl.: C07D 277/10, C11B 9/00, A61K 7/46, A23L 1/226

(54) **4-Alkanoyl-3-thiazoline und deren Verwendung als Riech- und Aromastoffe**
4-Alkanoyl-3-thiazolines and their use as fragrances and flavourings
4-Alkanoyl-3-thiazolines et leur utilisation comme parfums et arômes

(30) Priorität: 12.07.1999 DE 19932494
(43) Veröffentlichungstag der Anmeldung: 17.01.2001
(73) Patentinhaber: HAARMANN & REIMER GMBH, 37601 Holzminden (DE)
(72) Erfinder: Güntert, Matthias, Dr., Ridgewood, NJ 07450 (US); Lambrecht, Stefan, Dr., 37603 Holzminden (DE); Engel, Wolfgang, 80538 München (DE); Schieberle, Peter, Prof. Dr., 85354 Freising (DE)
(74) Vertreter: Mann, Volker, Dr.

(56) Entgegenhaltungen:
- CH-A- 565 515
- US-A- 3 778 518

## Beschreibung

Die Erfindung betrifft neue substituierte 3-Thiazoline, Verfahren zu ihrer Herstellung und ihre Verwendung als Aroma für Nahrungs- und Genußmittel.

In der Aromenindustrie besteht nach wie vor ein starker Bedarf an Verbindungen, die Nahrungs- und Genußmitteln einen olfaktorischen Eindruck vermitteln, wie er bei der thermischen Behandlung während des Koch-, Back- und Röstprozesses von Lebensmitteln entsteht. Die hierbei entstehenden aromatisierenden Verbindungen weisen vor allem röstige Noten auf. Solche Verbindungen sind bislang nur wenig für den technischen Einsatz verfügbar.

Die wichtigste Reaktion, die bei der thermischen Behandlung von Lebensmitteln verläuft, ist die Reaktion zwischen reduzierenden Zuckern und Aminosäuren, die sogenannte Maillard-Reaktion. Es entstehen während dieser Maillard-Reaktion insbesondere Aromastoffe aus der chemischen Substanzklasse der Heterocyclen. Diese Verbindungen enthalten ein oder mehrere Heteroatome, verschiedene Seitenketten und sind aromatisch oder teilweise hydriert (P.A. Finot, H.U. Aeschbacher, R.F. Hurrell, R. Liardon, *The Maillard Reaction in Food Processing, Human Nutrition and Physiology,* Birkhäuser Verlag, Basel, 1990).

In der Substanzklasse der 2-Thiazoline ist insbesondere das 2-Acetyl-2-thiazolin ein bekannter und industriell verwendeter Aromastoff, der aufgrund seiner Aromaeigenschaften vor allem dort eingesetzt wird, wo röstige Geschmackseigenschaften gewünscht werden (US-A 3 778 518). So wird 2-Acetyl-2-thiazolin zum Beispiel für Huhnaromen eingesetzt, wo es die typische Röstnote vermittelt. Der Schwellenwert für dieses 2-Thiazolin wird in der Literatur mit 0,016 - 0,022 ng/l in Luft angegeben (M. Rychlik, P. Schieberle, W. Grosch, *Compilation of Odor Thresholds, Odor Qualities and Retention Indices of Key Food Odorants,* Deutsche Forschungsanstalt für Lebensmittelchemie und Institut für Lebensmittelchemie der Technischen Universität München, Garching, 1998). Unter dem Schwellenwert versteht man die niedrigste Konzentration, bei der eine Verbindung sensorisch wahrgenommen werden kann.

Es wurden 4-Alkanoyl-3-thiazoline der Formel 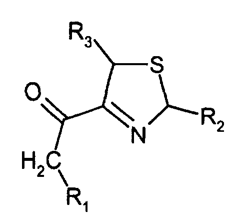 gefunden, in der R₁, R₂ und R₃ gleich oder verschieden sind und Wasserstoff oder Niederalkyl bedeuten.

Niederalkyl bedeutet einen geradkettigen oder verzweigten Kohlenwasserstoff mit 1 bis 3, insbesondere bevorzugt 1 oder 2 Kohlenstoffatome.

Die erfindungsgemäßen 3-Thiazoline, insbesondere das 4-Acetyl-3-thiazolin, haben überraschenderweise sehr niedrige Schwellenwerte. Beispielsweise hat das erfindungsgemäße 4-Acetyl-3-thiazolin (R₁,R₂,R₃=H) einen Schwellenwert, der bei 0,005 ng/l in Luft liegt. Dieser Schwellenwert ist damit deutlich niedriger als bei 2-Acetyl-2-thiazolin. Die Verbindung 4-Acetyl-3-thiazolin gehört damit zu den aromastärksten Verbindungen.

Das 4-Acetyl-3-thiazolin konnte in einem Reaktionsgemisch von Fructose und der Aminosäure 4-Carboxy-3-thiazolidin identifiziert werden (Beispiel 1).

Die Identifizierung gelang durch Auftrennung des Extraktes aus dem Reaktionsgemisch mittels multidimensionale Gas-Chromatogaphie und anschließender massenspektrometrischer Untersuchung. 4-Acetyl-3-thiazolin konnte durch Vergleich mit den analytischen Daten einer authentischen Probe eindeutig identifiziert werden.

Darüberhinaus wurden auch systematische Versuche mit einer chromatographischen Technik durchgeführt, die als Gaschromatographie-Olfaktometrie (GC-O) bezeichnet wird. Dabei werden die während des Chromatographievorgangs aufgetrennten Verbindungen am Ende der Trennkapillare mit der Nase einzeln abgerochen.

Mit Hilfe dieser Techniken konnten die geruchlichen und geschmacklichen Qualitäten von 4-Acetyl-3-thiazolin bestimmt werden.

4-Acetyl-3-thiazolin riecht und schmeckt nach Popcorn und Brotkruste und hat starke röstige Aromaeigenschaften.

Die Struktur der erfindungsgemäßen 3-Thiazoline wurde durch Vergleich mit synthetisch hergestelltem 4-Acetyl-3-thiazolin bewiesen. 4-Acetyl-3-thiazolin kann ausgehend von Diacetyl hergestellt werden. Diacetyl wird zunächst bromiert. Das entstandene Bromdiacetyl wird mit Natriumhydrogensulfid in 1-Mercaptodiacetyl überführt. Nach Reaktion mit Formaldehyd- und Ammoniak-Lösung sowie chromatographischer Reinigung wird 4-Acetyl-3-thiazolin erhalten (Beispiel 2).

Die erfindungsgemäßen Verbindungen eignen sich wegen ihres herausragenden organoleptischen Charakters vorzüglich als Riech- und Aromastoffe für den Einsatz in Aromakompositionen und Reaktionsaromen. Besonders überraschend ist, daß das 4-Acetyl-3-thiazolin den betreffenden Kompositionen eine sehr intensive popcornartige, röstige Note in ausgesprochen niedrigen Konzentrationen verleiht.

In Aroma-Kompositionen liegt die eingesetzte Menge der erfindungsgemäßen Verbindung vorzugsweise zwischen 0,0005 und 1 Gew.-%, insbesondere zwischen 0,001 und 0,5 Gew.-%, bezogen auf die gesamte Komposition. Derartige Aroma-Kompositionen können im gesamten Nahrungs- und Genußmittelbereich verwendet werden. Insbesondere sind sie geeignet für die Aromatisierung von Snacks, Suppen, Saucen, Fertiggerichten, Fettmassen, Backwaren, Joghurt, Speiseeis und Süßwaren. Die Dosierung derartiger Aroma-Kompositionen liegt vorzugsweise bei 0,005 bis 2 Gew.-%, insbesondere zwischen 0,01 und 1 Gew.-% bezogen auf das fertige Lebensoder Genußmittel.

Die Aromen können in flüssiger oder sprühgetrockneter oder verkapselter Form eingesetzt werden. Während sie in flüssiger Form in einem praxisüblichen Lösungsmittel wie Ethanol, Propylenglycol, Pflanzenöltriglyceriden oder Triacetin eingesetzt werden, gewinnt man die trockenen Aromen durch Sprühtrocknung oder durch Verkapselung nach einem der in der Aromenindustrie üblichen Verfahren. Dies sind insbesondere die Extrusion und die Sprühgranulation.

### Beispiele

### Beispiel 1: Modellreaktion Fructose und 4-Carboxy-3-thiazolidin

1,8 g Fructose und 0,44 g 4-Carboxy-3-thiazolidin wurden in 2,1 g Wasser gelöst und mit 19,1 g Kieselgel verrieben. Diese Mischung wurde für 10 min bei 150°C auf einem Aluminium-Block unter Luftatmosphäre erhitzt. Nach dem Abkühlen wurde mit Diethylether extrahiert. Die organische Pase wurde mit Natriumhydrogencarbonat-Lösung und mit Natriumchlorid-Lösung gewaschen, über Natriumsulfat getrocknet und aufkonzentriert.

Das Reaktionsgemisch wurde mit Säulenchromatographie an Kieselgel fraktioniert. Die einzelnen Fraktionen wurden am Riechgaschromatographen abgerochen, durch multidimensionale Gas-Chromatographie getrennt und anschließend durch GC/MS untersucht. 4-Acetyl-3-thiazolin konnte durch Vergleich mit den analytischen Daten einer authentischen Probe eindeutig identifiziert werden

### Beispiel 2: Herstellung von 4-Acetyl-3-thiazolin

10 mmol α-Bromcarbonylverbindung (hergestellt durch Bromierung der entsprechenden α-Dicarbonylverbindung nach P.Ruggli, M.Herzog, J.Wegmann, H.Dahn, *Helv. Chim. Acta*, 1946, 29(1), 95-101 bei Verwendung der halben Brommenge) werden bei 0°C zu einer Lösung von 30 mmol Natriumhydrogensulfid in 20 ml 5%iger Natronlauge getropft. Anschließend erwärmt man auf Raumtemperatur und läßt eine Stunde rühren. Dann wird mit 20%iger Zitronensäure auf pH 3 angesäuert und die Mercaptodiketoverbindung mit Ether extrahiert.

Diese Etherphase wird mit 10 mmol Aldehyd (Formaldehyd als wäßrige, 35%ige Lösung) und mit 10 mmol Ammoniak-Lösung (wäßrig, 25%ig) versetzt. Man rührt bei Raumtemperatur und verfolgt den Verlauf der Reaktion durch GC/MS.Nach 1-10 Stunden wird die Etherphase konzentriert und die Mischung durch Säulenchromatographie mit Pentan-Ether-Gradienten an Kieselgel gereinigt. Man erhält reine 4-Alkanoyl-3-thiazoline in Ausbeuten von 5-20%.

| Massenspektrum von 4-Acetyl-3-thiazolin | |
|---|---|
| m/z | Intensität |
| | % |
| 41 | 15 |
| 42 | 19 |
| 43 | 91 |
| 45 | 29 |
| 46 | 19 |
| 59 | 19 |
| 60 | 19 |
| 86 | 20 |
| 128 | 100 |
| 129 | 66 |

### Beispiel 3: Herstellung eines Röst-Aromas

Es wurden vermischt (alle Angaben in g):

| | |
|---|---|
| 3-Methylthiopropanal (1%ig in Pflanzenöltriglyceriden) | 1,0 |
| 2,3-Diethyl-5-methylpyrazin | 1,0 |
| Isoamylcaprylat | 1,0 |
| Diacetyl (10%ig in Triacetin) | 2,0 |
| 2-Methylbuttersäure | 5,0 |
| Isoamylalkohol | 10,0 |
| Delta-Dodecalacton | 10,0 |
| 2-Phenylethanol | 15,0 |
| 2-Methylbutanal | 20,0 |
| Caprylsäure (10%ig in Triacetin) | 25,0 |
| Dimethyloxyfuron (1%ig in Propylenglycol) | 100,0 |
| 2,5-Dimethyl-4-hydroxy-3(2H)-furanon (15%ig in Propylenglycol) | 500,0 |
| Pflanzenöltriglyceride | 9310,0 |
| Summe | 10000,0 |

Ersetzte man 0,1 - 0,5 g des Lösungsmittels Pflanzenöltriglyceride durch 0,1 - 0,5 g 4-Acetyl-3-thiazolin, so wurde das Aroma deutlich typischer in Richtung Popcorn und Brotkruste.

## Patentansprüche

1. 4-Alkanoyl-3-thiazoline der Formel 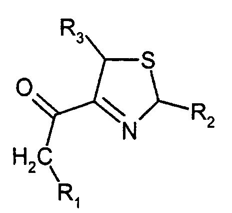 in der R₁, R₂ und R₃ gleich oder verschieden sind und Wasserstoff oder Niederalkyl bedeuten, wobei
Niederalkyl einen geradkettigen oder verzweigten Kohlenwasserstoff mit 1 bis 3 Kohlenstoffatomen bedeutet.

2. 4-Alkanoyl-3-thiazoline nach Anspruch 1, wobei R_{1,} R₂ und R₃ gleich oder verschieden sind und Wasserstoff, Methyl oder Ethyl bedeuten.

3. 4-Alkanoyl-3-thiazoline nach Anspruch 1, wobei es sich um 4-Acetyl-3-thiazolin handelt.

4. Aroma- und Riechstoff-Kompositionen enthaltend 4-Alkanoyl-3-thiazoline nach Anspruch 1.

5. Aroma- und Riechstoff-Kompositionen enthaltend 4-Alkanoyl-3-thiazoline nach Anspruch 2

## Claims

1. 4-alkanoyl-3-thiazolines of the formula 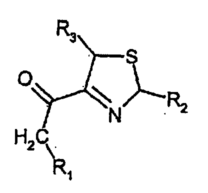 where R₁, R₂ and R₃, which are the same or different, represent hydrogen or lower alkyl, wherein lower alkyl represents a straight-chain or branched hydrocarbon containing 1 to 3 carbon atoms.

2. 4-alkanoyl-3-thiazolines according to claim 1, wherein R₁, R₂ and R₃, which are the same or different, represent hydrogen, methyl or ethyl.

3. 4-alkanoyl-3-thiazolines according to claim 1, wherein 4-acetyl-3-thiazoline is used.

4. Flavouring and perfume compositions containing 4-alkanoyl-3-thiazolines according to claim 1.

5. Flavouring and perfume compositions containing 4-alkanoyl-3-thiazolines according to claim 2.

## Revendications

1. 4-alcanoyi-3-thiazolines de formule 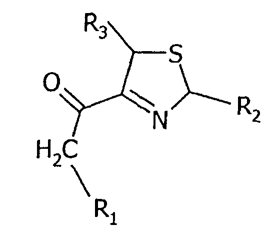 dans laquelle R₁, R₂ et R₃ ayant des significations identiques ou différentes représentent chacun l'hydrogène ou un groupe alkyle inférieur,
un groupe alkyle inférieur consistant en un radical hydrocarboné à chaîne droite ou ramifiée en C₁-C₃.

2. 4-alcanoyl-3-thiazolines selon revendication 1, pour lesquelles R₁, R₂ et R₃, ayant les significations identiques ou différentes, représentent chacun l'hydrogène, un groupe méthyle ou éthyle.

3. 4-aicanoyi-3-thiazoiine selon revendication 1 : la 4-acétyl-3-thiazoline.

4. Compositions d'arômes et de parfums contenant des 4-alcanoyl-3-thiazolines selon revendication 1.

5. Compositions d'arômes et de parfums contenant des 4-aicanoyl-3-thiazolines selon revendication 2.
